# EUROPEAN PATENT APPLICATION

(11) **EP 4 356 874 A1**
(43) Date of publication of application: **24.04.2024**
(21) Application number: 22306580.6
(22) Date of filing: 19.10.2022
(51) Int. Cl.: A61F 2/00

(54) **MESH DELIVERY TOOL AND METHODS ASSOCIATED THEREWITH**

(71) Applicant: Sofradim Production, 01600 Trevoux (FR)
(72) Inventor: DOUCET, Geneviève, 01480 Frans (FR)
(74) Representative: Germain Maureau

(57) **Abstract**

A mesh delivery tool comprising: a body defining a length and a width, the length extending longitudinally between a proximal end portion and a distal end portion with a central slotted portion positioned therebetween, the width extending transversely between a first side and an opposite second side, the distal end portion extending between the first side and the second side defining the width of the body, the distal end portion including a fixed side part and a free side part opposite the fixed side part, the fixed side part extending from the central slotted portion.

## Description

### Technical Field

The present disclosure relates to a mesh delivery tool configured to deliver a surgical mesh into a patient, and particularly a mesh delivery tool configured to deliver a self-gripping surgical mesh into a patient.

### Background

Conventional surgical mesh have been used for the treatment of many types of soft tissue defects, such as hernias and/or prolapse. For example, in some instances, surgical mesh may be designed as a wall reinforcement prosthesis, and particularly an abdominal wall reinforcement prosthesis, intended to treat a hernia by temporarily or permanently filling or covering a tissue defect. Surgical mesh are generally made from one or more biocompatible yarns or filaments formed into a mesh body or textile having any number of shapes, for example rectangular, circular or oval, depending on the anatomical structure to which they are intended to adapt. Some of these mesh are made from entirely bioresorbable yarns or filaments and are intended to disappear after having performed their reinforcing role while dell colonization takes place and tissue rehabilitation takes over. Other mesh include non-bioresorbable yarns or filaments and are intended to remain permanently in the body of the patient. The yarns or filaments of the mesh are often knitted, braided and/or woven together and locked into place with each other. For example, a knit mesh commonly includes yarns or filaments knitted together in at least a warp and weft direction such that the warp and weft yarns are interwoven with each other to ensure the warp and weft yarns are locked into a position relative to each other to form and maintain the overall structure of the knit mesh.

In some instances, the surgical mesh may further include a plurality of gripping members (e.g., barbs, spiked naps, hooks, arrows, etc.) configured to attach directly to biological tissue (and/or other portions of the mesh when folded or rolled) forming a self-gripping surgical mesh. The gripping members are intended to help to attach the surgical mesh to biological tissues, such as muscle and fascia, limiting the migration of the mesh following proper implantation. By attaching directly to the biological tissue, the need to use additional surgical fasteners (e.g., sutures, staples, glue, tacks, etc.) is at least minimized, if not eliminated, thereby limiting damage to the tissue inflicted by the fastener, reducing the formation of scar tissue around the fastener, and reducing a patient's pain often associated with the use of surgical fasteners. The gripping members may be formed from one or more biocompatible yarns or filaments. Some non-limiting examples of self-gripping mesh are described in U.S. Patent Nos. 6,596,002; 7,331,199; 9,186,235.

As the grip members are designed to grip to surrounding tissue (and/or other portions of a mesh), some medical personnel, and particularly surgeons, may experience difficulty manipulating a self-gripping mesh during introduction without prematurely attaching to any surrounding biological tissue. It may also be difficult to unfold or unroll a self-gripping mesh without the grip members attaching to overlapping portions of the mesh when folded or rolled.

It would be an object of the present application to provide a mesh delivery tool designed to facilitate the introduction of any conventional surgical mesh, and particularly a self-gripping mesh, into a patient while avoiding premature contact with the biological tissue. By avoiding premature tissue contact, the delivery tool may also provide the ability to position and reposition as many times as needed to determine a final position to implant the mesh.

It would further be an object of the present application to provide a mesh delivery tool designed to fold any conventional surgical mesh, and particularly a self-gripping mesh, into a folded configuration which limits or avoids attachment or snagging of any overlapping mesh portions.

It would further be an object of the present application to provide a mesh delivery tool useful in the treatment of inguinal hernias, and particularly the introduction of a surgical mesh through the tissue defect and/or around the spermatic cord.

### SUMMARY

The present disclosure describes a mesh delivery tool configured to deliver a mesh into a patient for the repair and/or treatment of a soft tissue defect, such as a hernia, and particularly an inguinal hernia.

The mesh delivery tool is configured to introduce a mesh in a folded and/or overlapping configuration into a patient while limiting, if not avoiding completely, any overlapping mesh portions from attaching to each other.

The mesh delivery tool is configured to deliver a self-gripping mesh into a patient while limiting, if not avoiding completely, the mesh from attaching to biological tissue prematurely.

In embodiments, the mesh delivery tools described herein may include a body defining a length and a width, the length of the body (and/or tool) extends longitudinally between a proximal end portion and a distal end portion with a central slotted portion positioned therebetween. The width of the body (and/or tool) extends transversely between a first side and an opposite second side. The distal end portion extends between the first and second sides, and particularly from the first side to the second side, defining the width of the body (and/or tool). The distal end portion includes a fixed side part extending from the central slotted portion and a free side part opposite the fixed side part (and/or the central slotted portion).

The proximal end portion may extend generally longitudinally at a location positioned between (and including) a center of the width of the body (and/or tool) and either the first side or the second side of the body (and/or tool), whichever is opposite the free side part of the distal end portion and/or the same side as the fixed side part of the distal end portion. In some embodiments, the proximal end portion is located on or near the center of the width of the body (and/or tool). In some embodiments, the proximal end portion is off-set from the center of the width of body (and/or tool) and closer to the fixed side part than the free side part of the distal end portion.

The central slotted portion extends on a proximal part from the proximal end portion and is connected on a distal part to the fixed side part of the distal end portion. In some embodiments, the proximal part of the central slotted portion is a bent and/or curved rod extending from the proximal end portion towards the first side of the body, and the distal part is straight rod extending longitudinally along or near the first side of the body.

The central slotted portion includes at least a central rod and at least one free central arm, each free central arm separated from the central rod by a slot configured to receive and/or maintain a portion of a surgical mesh therein. In some embodiments, central slotted portion includes two central arms and two slots, each arm and slot pair positioned on opposite top or bottom surfaces of the central rod.

The central rod, the free arm, and the slot are configured to receive and/or maintain a portion of the mesh within the slot. In some embodiments, the central rod, the free arm, and the slot are configured to receive a portion of the mesh within the slot and further configured to be pressed or squeezed together to maintain the mesh therebetween.

In some embodiments, the one or more slots of the central slotted portion may extend only through a part, if not all, of the central slotted portion of the body.

In some embodiments, the one or more slots of the central slotted portion may extend through the central slotted portion and at least a part of one of the proximal end portion, the distal end portion, or both.

In some embodiments, the distal end portion is a bent rod. In some embodiments, the bent rod is curved and/or elliptical. In some embodiments, the bent rod includes a plurality of intersecting straight rods defining at least a partial polygonal shape. In some embodiments, the bent rod further includes a tab member extending therefrom.

The overall shape of the body (and/or tool) may be generally a question mark shape, a mirror image of a question mark shape, or a sickle shape.

The mesh delivery tools described herein may be configured as a right-handed tool, a left-handed tool, or both.

In some embodiments, the mesh delivery tools described herein may be monolithic and/or made from a single piece of material. In some embodiments, the mesh delivery tools described herein may be made from two or more removable and/or interchangeable pieces of material.

The mesh delivery tools described herein may further include a hinge located proximal the one or more slots of the central slotted portion. The hinge configured to allow the central rod and/or one or more free central arms to pivot relative to each other between an open and closed configuration.

Surgical kits are also described including at least one of any of the mesh delivery tools described herein. The kit may further include an implantable surgical mesh independent of the delivery tool and/or combined with the delivery tool. The implantable surgical mesh may include one or more of a slit extending to an outer periphery of the mesh, a plurality of grip members extending from at least one face of the mesh, or both.

Methods of repairing or treating a soft tissue defect are also provided.

### BRIEF DESCRIPTION OF THE DRAWINGS

Various embodiments are described herein with reference to the drawings wherein:
Fig. 1A is a top view of a mesh delivery tool as described in one or more embodiments herein;
Fig. 1B is a perspective side view of the tool of Fig. 1A as described in one or more embodiments herein;
Fig. 1C is a cross-sectional side view of the tool of Fig. 1A as described in one or more embodiments herein;
Figs. 2A and 2B are each generally a top view of a combined mesh-mesh delivery tool as described in one or more embodiments herein;
Figs. 3-5 are each a top view of a mesh delivery tool as described in one or more embodiments herein;
Figs. 6-8 are each a cross-sectional side view of a mesh delivery tool as described in one or more embodiments herein;
Figs. 9-10 are each a cross-sectional side view of a central slotted portion of a mesh delivery tool as described in one or more embodiments herein;
Figs.11A-11G are each a top view of a distal end portion of a mesh delivery tool as described in one or more embodiments herein;
Figs. 13A and 13B are a top view and a cross-sectional side view, respectively, of a mesh delivery tool as described in one or more embodiments herein; and
Figs. 14A-14F schematically illustrate various steps of a method for repairing an inguinal hernia using a mesh delivery tool as described in one or more embodiments herein.

### DETAILED DESCRIPTION

The present disclosure describes mesh delivery tools configured to be secured to and/or carry an implantable surgical mesh to a site of implantation inside a patient to treat a soft tissue defect, and particularly for the treatment of hernia or inguinal hernia.

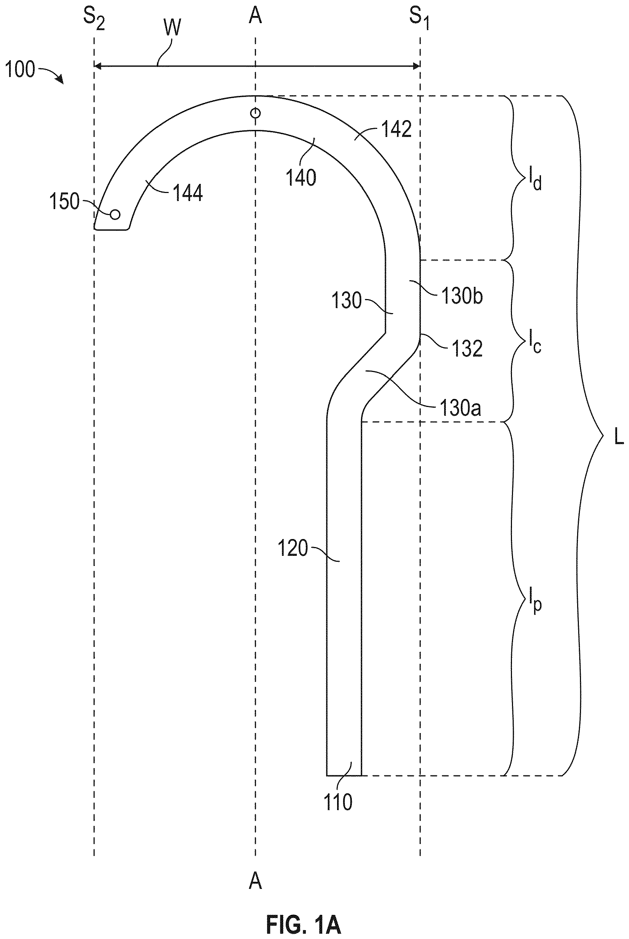

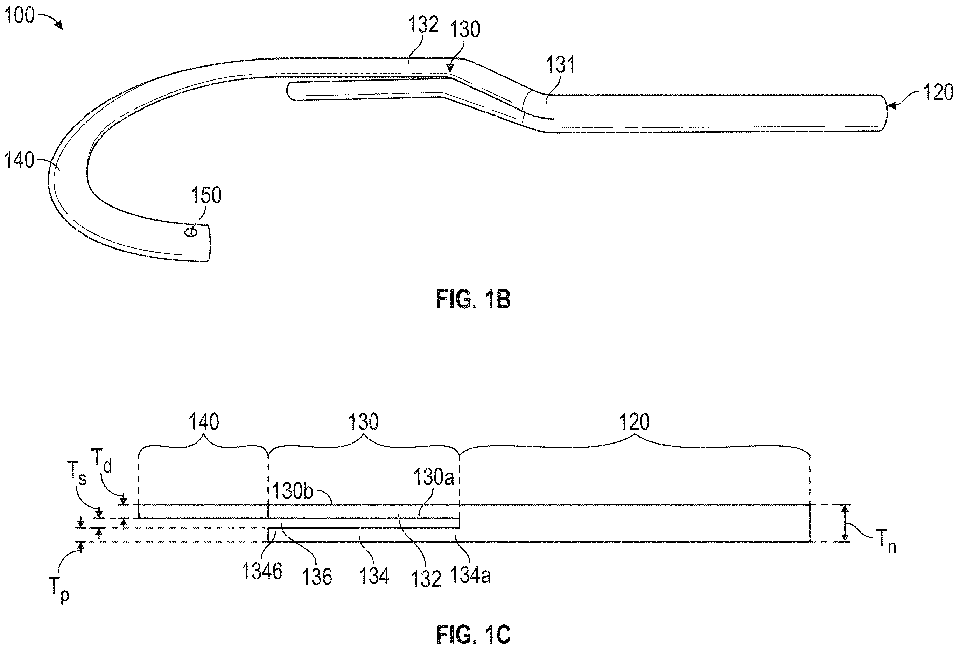

Figs. 1A-1C depict a mesh ancillary or tool 100 for mesh delivery, and particularly delivery of an implantable surgical mesh including a slit and/or a plurality of grip members. The tool 100 includes a body 110 defining a length L and a width W, the length L extending longitudinally between a proximal end portion 120 and a distal end portion 140 with a central slotted portion 130 positioned therebetween. The width W extends transversely to the length between a first side S₁ and an opposite second side S₂. The distal end portion 140 includes a fixed side part 142 fixed to the central slotted portion 130 and a free side part 144 opposite the fixed side part 142. The distal end portion 140 extends between the first side S₁ and the second side S₂ defining the width W of the tool 100. In some embodiments, the distal end portion 140 is curved between the fixed 142 and free sides 144.

The proximal end portion 120 may define a generally straight rod. The straight rod may extend along a longitudinal axis of the tool 100 at a location off-set from a center A of the width W, and particularly closer to the first side S₁ of the body 110 than the second side S₂. In some embodiments, the proximal end portion 120 may be closer to the fixed side part 142 of the distal end portion 140 than the free side part 144 of the distal end portion 140. The proximal end portion 120 may be located anywhere between the center A of the width W and the first side S₁. The proximal end portion 120 defines a length lₚ. In some embodiments, the proximal end portion 120 represents a handle portion for the mesh delivery tool 100.

As further illustrated in Figs. 1A-1C, the central slotted portion 130 may define a bent rod extending from a distal part of the proximal end portion 120 to the fixed side part 142 of the distal end portion 140. The central portion 130 may include a bent or curved proximal part 130a extending from the distal part of the proximal end portion 120 towards the first side S₁ and a straight distal part 130b extending generally longitudinally (and/or parallel to the longitudinal axis A of the proximal end portion) along the first side S₁.

The central slotted portion 130 may include a central rod 132 and a central arm 134 separated by a slot 136 configured to receive and/or maintain at least a portion of the mesh in a folded configuration. The central rod 132 connects the proximal end portion 120 to the distal end portion 140 of the body 110. The central arm 134 includes a proximal part 134a and a distal part 134b, the proximal part 134a extending distally from the distal part of the proximal end portion 120 of the body 110 and/or a proximal part 132a of the central rod 132. The distal part 134b of the central arm 134 being free of the rest of the body 110. The central rod 132, the slot 136, and central arm 134 being vertically aligned.

In some embodiments, the central arm 134 (and/or the slot 136) may be configured to receive a portion of a mesh therein but not maintain the mesh therein without pressing upon and/or squeezing the central arm 134 towards the central rod 132 thereby narrowing or closing the slot 136 and pinching the mesh therebetween. Because the arm 134 includes a free end, the arm 134 may display a natural ability to flex or bias sufficiently towards or away from the central rod 132 as needed.

In some embodiments, the central arm 134 and central rod 132 (and/or slot 136) may be configured to both receive and maintain a portion of a mesh therein without having to apply pressure thereto.

In some embodiments, the slot 136 extends at least through the central slotted portion 130 of the body 110. In some embodiments, the slot 136 extends only through the central slotted portion 130 of the body 110.

As further depicted in Figs. 1A-1C, distal end portion 140 may define a bent rod 141 extending transversely between the first side S₁ and the second side S₂ of the body 110. In some embodiments, the bent rod 141 is a curved or elliptical bent rod. However, as provided in additional non-limiting examples hereinbelow, the bent rod 141 may define other configurations as well.

The distal end portion 140 may also include one or more fastener apertures 150 defined therethrough, the fastener apertures 150 configured to allow a suture or other fastener such as a string, filament, thread, staple, pin, yarn, glue, etc. to be passed therethrough to secure a portion of a mesh to the body 110, and particularly the distal end portion 140 of the body 110.

As depicted particularly in Fig. 1A, the length L of the body 100 is equal to the combination of a length lₚ of the proximal end portion 120, a length l_{c} of the central slotted portion 130, and a length l_{d} of the distal end portion 140. In some embodiments, lₚ≥l_{c}≥l_{d}.

In some embodiments, the length lₚ of the proximal end portion 120 may range from: about 1 to about 20cm; about 2 to about 15 cm; or about 5 to about 10cm. In some embodiments, the length l_{c} of the central slotted portion 130 may range from: about 0.5 to about 15cm; about 1 to about 12 cm; or about 2 to about 10cm. In some embodiments, the length l_{d} of the distal end portion 140 may range from: about 0.25 to about 12cm; about 0.5 to about 10 cm; or about 1 to about 7cm.

As best shown in Fig. 1C, the distal end portion 140 may define a thickness T_{d} generally equal to a thickness T_{c} of the central rod and/or the central arm Tₐ. In some embodiments, the thickness T_{d} of the distal end portion 140 (or the thickness T_{c} of the central rod 132), the thickness Tₛ of the slot 136, and the thickness Tₐ of the central arm 134 may equal a thickness Tₚ of the proximal end portion 120.

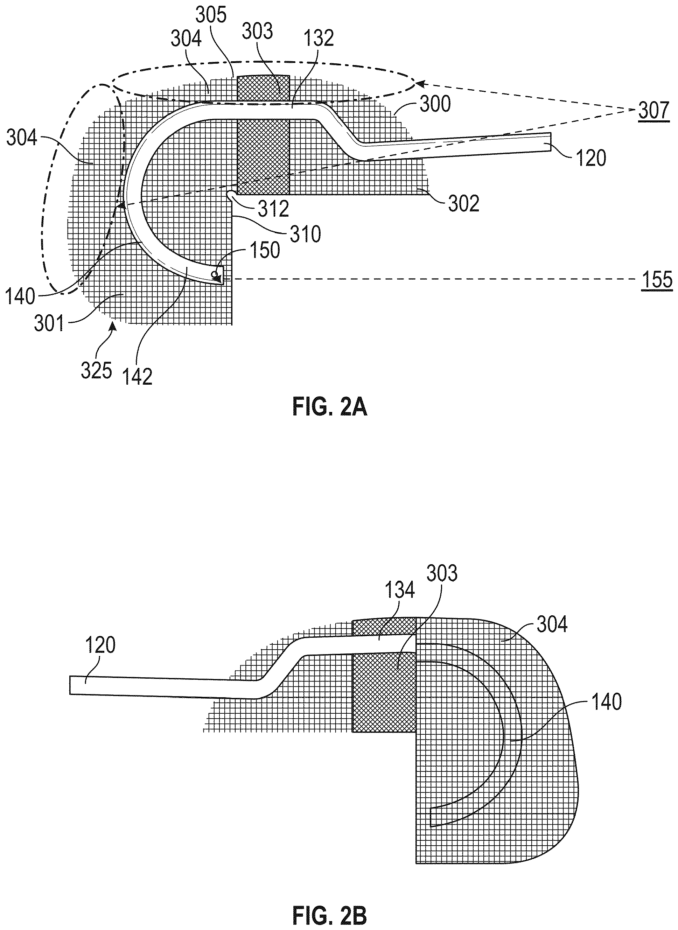

Figs. 2A and 2B depict the tool 100 of Figs. 1A-1C in combination with an implantable surgical mesh 300. The mesh 300 is defined by an outer peripheral edge 305 and may include a slit 310 connecting a mesh opening 312 to the peripheral edge 305. The slit 310 generally separating a first half 301 of the mesh 300 from a second half 302 of the mesh 300. The mesh 300 may be self-gripping in that it includes a plurality of grip members 325 extending from at least one face thereof, the grip members 325 configured to attach directly to tissue, directly to other overlapping portions of the mesh, and/or a second mesh. The mesh 300 may include a flap 303 attached thereto and designed to extend across the slit 310 when implanted. The flap may or may not include grip members.

As shown in Fig. 2A, the distal end portion 140 of the delivery tool 100 is configured to support and/or be positioned on a first half 301 of the mesh 300 in an unfolded or generally flat configuration. As the distal end portion 140 is located on the same side as the grip members 325 of first half 301 of the mesh 300, at least the flat portion 301 of the mesh 300 and the grip members 325 extending therefrom are configured to be separated from the biological tissue by the distal end portion 140 during delivery, thereby reducing or limiting the likelihood of premature gripping of the mesh 300 to the biological tissue.

As further shown in Fig. 2A, at least the central slotted portion 130 is configured to receive and/or maintain at least a portion of the second half 302 of the mesh 300 in a folded or overlapping configuration along one side of the slit 310. The folded or overlapping mesh pieces being located within the slot 136 between the central rod 132 and the free central arm 134. A flap 303 may also be folded and received/maintained in the slotted portion as well.

The mesh 300, and particularly, the portion of the mesh in a flat or unfolded configuration 301, may be secured to the distal end portion 140 via a fastener 155 secured to both the mesh 300 and the body 110 through fastener aperture 150.

As further demonstrated in Figs. 2A-2B, the proximal end portion 120 of the body 110 is located closer to the first side S₁ (and/or opposite the free side part 144 of the distal end portion 140) than the second side S₂ (and/or the fixed side part 142 of the distal end portion 140). By being located at a position generally between a center A of the width W of the body (or tool) and the first side S₁ (and/or a side opposite the free side part 142 of the distal end portion 140), the proximal end portion or handle 120 does not interfere with the folding of the mesh 300, and particularly folding the second half 302 of the mesh 300 in half longitudinally.

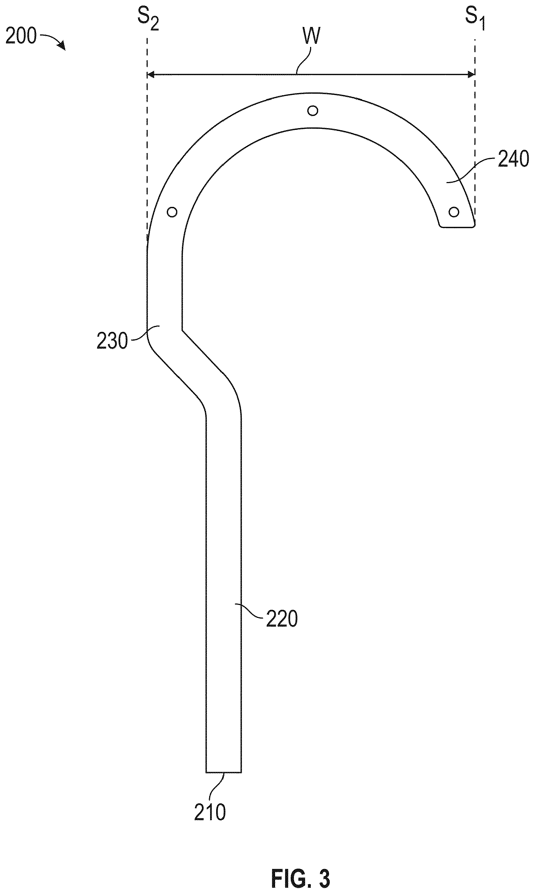

In addition, since the mesh delivery tool 100 can be spaced from the outer peripheral edge 305 of the mesh 300, including the unfolded 301 and/or the folded 302 portions, an outer periphery 304 of the mesh 300 may extend between the tool 100 and the outer peripheral edge 305 as highlighted by the ovals of Fig. 3A. This feature provides medical personnel with an opportunity to adjust, e.g., trim, the size and/or shape of the mesh 300 along the outer periphery 304, while the mesh 300 is retained in the tool 100 and prior to implantation. Also, since the slit 310 and the mesh aperture 312 are also spaced inwardly from the tool 100, medical personnel can also adjust, e.g., trim/cut, the size and/or shape of slit 310 and opening 312, as needed, to properly accommodate a certain tissue or organ, such as the spermatic cord, prior to implantation.

In some embodiments, as shown in at least Figs. 1A-1C, the body 110 (and/or tool 100) may be a right-handed body 110 (and/or tool 100). However, the body 110 (and/or tool 100) may also be formed as a left-handed body 210 (and/or tool 200) as shown in Fig. 3. Essentially, the left-handed body 210 (and/or tool 200) is the mirror-image of the right-handed body 110 (and/or tool 100) of Fig. 1A. Particularly, the left-handed body 210 (and/or tool 200) defines a length L and a width W, the length L extending longitudinally between a proximal end portion 220 and a distal end portion 240 with a central slotted portion 230 positioned therebetween. The width W extends transversely between a first side S₁ and an opposite second side S₂. The distal end portion 240 includes a fixed side part 242 fixed to the central slotted portion 230 and a free side part 244 opposite the fixed side part 242. The distal end portion 240 extends between the first side S₁ and the second side S₂, and particularly from the second side S₂ to the first side Si, defining the width W.

In some embodiments, the body (and/or tool) may generally define a question mark shape (see at least Figs. 1A-1C). In some embodiments, the body (and/or tool) may generally define a mirror-image of a question mark shape (see Fig. 3).

Although the configurations described herein may be available in a right-handed or left-handed version, the remaining Figures may illustrate only one configuration for clarity purposes and/or brevity but are not intended to be limited to only the version depicted and are intended to be suitable for a right handed tool, a left handed tool, or both.

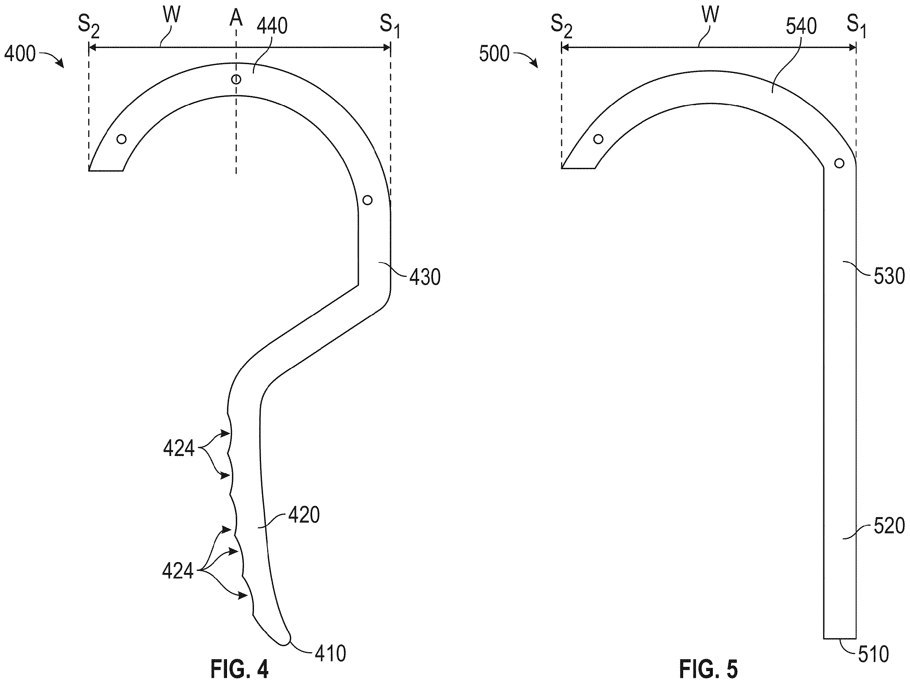

In Figs. 4 and 5, each tool 400, 500, respectively, is similar to the tool of Figs. 1A-1C, including a proximal end portion or handle 420, 520, a central slotted portion 430, 530, and a distal end portion 440, 540, however the alignment or location of the proximal end portion 420, 520 is different. For example, in some embodiments as shown in Fig. 4, the proximal end portion 420 may be generally located on and/or near the center A of the width W of the body 410. While in some embodiments as shown in Fig. 5, the proximal end portion 520 (and the central slotted portion) may be located only along the first side S₁.

In some embodiments, as further shown in Fig. 4, the handle portion 420 may display a slight bend or curve to facilitate a bit of initial tension to the tool 400 prior to loading of the mesh, the tension helpful in releasing of the mesh at the site of implantation. In some embodiments, as further shown in Fig. 4, the handle portion 420 may include or be covered with an anti-slip material or design, such as a plurality of finger grips 424 either formed therein or made of a separate rubber material and slid thereon.

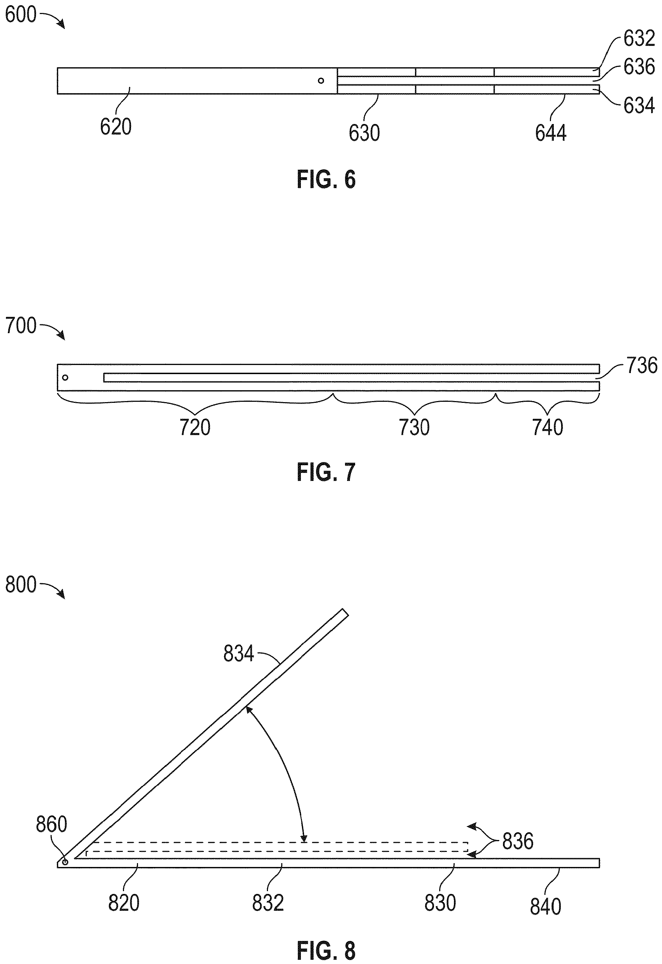

In Figs. 6-8, each tool 600, 700, 800 respectively, again is generally similar to the tool of Figs. 1A-1C, including a proximal end portion or handle 620, 720, 820, a central slotted portion 630, 730, 830, and a distal end portion 640, 740, 840 however the slot 636, 736, 836 may extend beyond just the central portion 630, 730, 830. For example, in some embodiments as shown in Fig. 6, the slot 636 may further extend along at least a portion, if not all, of the distal end portion 640 dividing both the central 630 and distal end portions 640 into two generally parallel rods/arms 632, 634 fixed to the proximal end portion 620 and free on the distal end portion 640. While in some embodiments as shown in Fig. 7, the slot 736 may further extend along at least a portion, if not all, of the proximal end portion 720. Although depicted as extending through the distal end portion 740 in Fig. 7, in some embodiments as represented in Fig. 8, the slot 836 may only extend from a part of the proximal end portion 820 to the central portion 830 and not the distal end portion 840.

As further illustrated in Fig. 8, in some embodiments, the slot 836 may split the proximal end portion 820 and the central portion 830 into two generally parallel rods/arms 832, 834 and the body 810 may further include a hinge 860 positioned proximal the slot 836. The hinge 860 configured to pivot the rod 832 and arm 834 ( as indicated by the arrow) between an open configuration (shown in Fig. 8) wherein the slot 836 is expanded to easily receive the mesh but not necessarily maintain the mesh therein and a closed configuration (as indicated in phantom) wherein the slot 836 is narrowed (and the rod and arm are generally parallel to each other) to maintain the mesh therein.

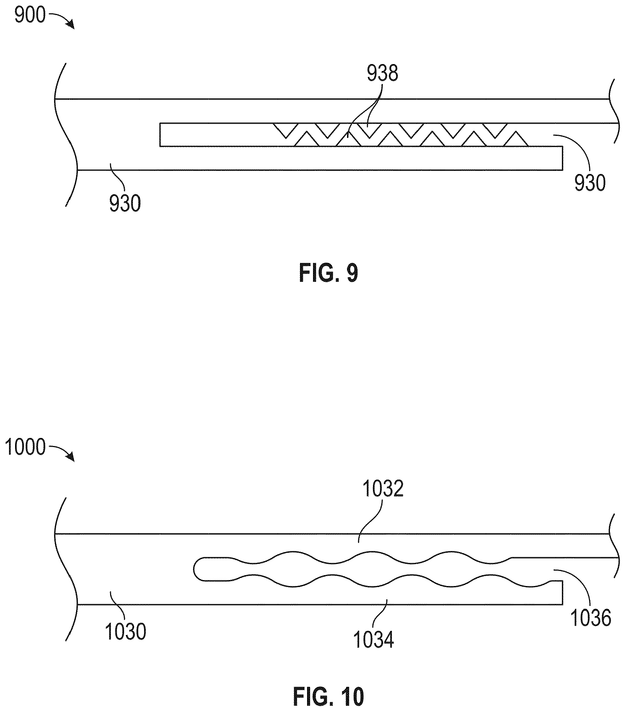

Figs. 9 and 10 focus on at least a central slotted portion 930, 1030 of a tool 900, 1000 as described herein. As shown in Fig. 9, in some embodiments, the slot 936 may not be smooth. For example, in some embodiments, at least a part of the slot 936 may further include a textured or micro-textured surface 938 designed to improve the ability of the slot 936 to maintain the mesh therein. In some embodiments, the texture or micro-texture (e.g., bumps, ridges, channels, etc.) may shaped and sized to fit within pores defined in the mesh. In some embodiments, the texture or micro-texture surface 938 may be formed by etching, grinding, sanding, molding, pressing, etc.

In Fig. 10, the slot 1036 separates at least the central portion 1030 into two generally parallel rod/arms, and particularly a central rod 1032 and a central arm 1034, wherein the surface of the slot is not simply planar or a straight edge, but rather is non-linear including a bent, curved, and/or sinusoidal edge to improve the ability of the slot to maintain the mesh therein.

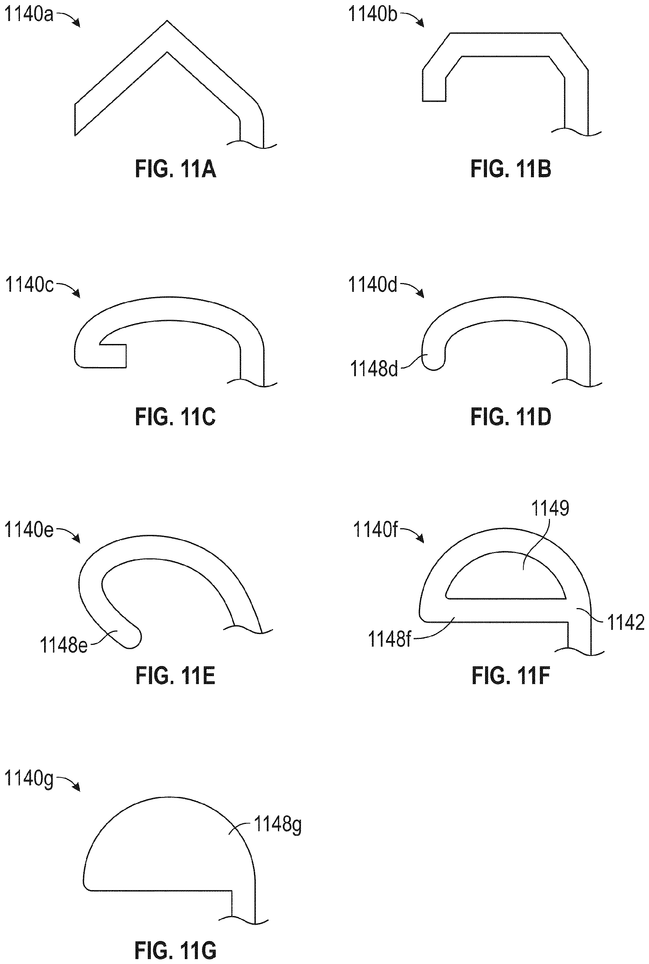

Figs. 11A-11F illustrate various designs of suitable distal end portions of the tools described herein. The proximal end portions and the central slotted portions are not shown but may as described in any of the embodiments herein. For example, in Figs. 11A and 11B, in some embodiments, the distal end portion 1140a, 1140b, respectively, may define a bent configuration including a plurality of intersecting straight rods representing at least part of any suitable polygonal design, such as a triangular or octagonal design, respectively.

In Figs. 11C-11G, the bent distal end portion 1140c-e is curved, similar to the tool of Figs. 1A-1C, however in Figs. 11C-11E, the distal end portion 1140c-e may include a tab member 1148c-e extending either transversely back towards the first side S₁ (and/or fixed side part 1142), longitudinally towards the proximal end portion 1120, or both, respectively.

In Figs. 11F-11G, in some embodiments, the tab member 1148f-g may extend transversely completely across the width W to the fixed side part 1142 (and/or central portion 1130). In Fig. 11F, the distal end portion 1140f may define a frame including a distal end aperture 1149. In some embodiments, the frame generally defines a half-circle or half-oval shape. In Fig. 11G, the distal end portion 1140g may form a wall 1148g without an aperture, the wall 1148g configured to cover a majority of the unfolded portion of the mesh when combined therewith.

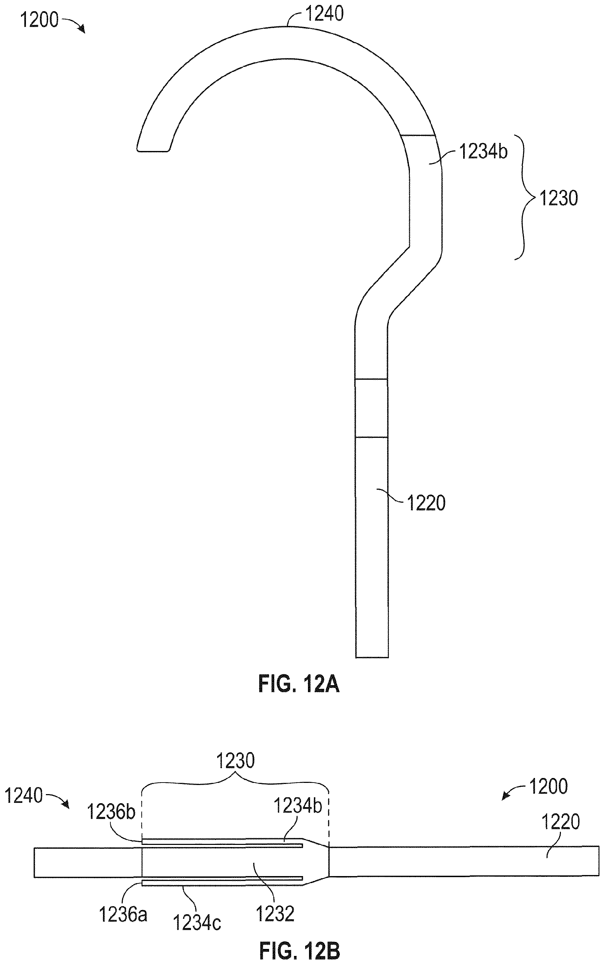

In some embodiments, instead of the tool or body being a right-handed design or a left-handed design, the mesh delivery tool may be designed to be both. For example, as shown in Figs. 12A-12B, the tool 1200 is generally similar to the tool of Figs. 1A-1C, including a proximal end portion 1220, a central slotted portion 1230 and distal end portion 1240, however the central slotted portion 1230 includes a first and second slot 1236a, 1236b. The first slot 1236a separates a central rod 1232 from a first central free arm 1234a on a bottom side of the body 1210. The second slot 1236b separates the central rod 1232 from a second central free arm 1234b on a top side opposite the bottom side of the central rod 1232. The second free central arm 1234b, the second slot 1236b, and the central body rod 1232 may be vertically aligned with each other, as well as with the first free central arm 1234a and the first slot 1236a. By including a slot 1236a, 1236b on each of the opposite top and bottom sides of the body, the delivery tool 1200 can initially operate as a right-handed design and simply be flipped 180 degrees (prior to the addition of the mesh) to operate as a left-handed design.

The tools described herein and any portion thereof can be made of any suitable biocompatible and/or sterilizable materials. Some non-limiting examples include metals, such as stainless steel, silver, titanium, nitinol, and the like, as well as polymeric materials, such as polyether ether ketones (PEEK), polycarbonates, and the like, and combinations thereof.

The tools described herein and any portion thereof may be formed using any suitable method known to those of ordinary skill. Some non-limiting examples of methods of forming the delivery tool include 3D-printing, extrusion, molding, casting, pressing, and the like. In some embodiments, the tools described herein may be made from 3D printing such as stereolithography (SLA), selective laser sintering (SLS), fused deposition modeling (FDM), digital light process (DLP), multi-jet fusion (MLF), polyjet, direct metal laser sintering (DMLS), and/or electron beam melting (EBM). Once formed, the tools described herein may be sterilized and/or packaged using any suitable method know to those of ordinary skill.

In some embodiments, the mesh delivery tools described herein may be made of a single monolithic structure. In some embodiments, the mesh delivery tools described herein may be made of two or more pieces of the same or different materials. For example, in some embodiments, the proximal end portion or handle portion may be made of a softer material for better feel when handled than the central or distal end portions which may be harder/stiffer to properly support the mesh thereon.

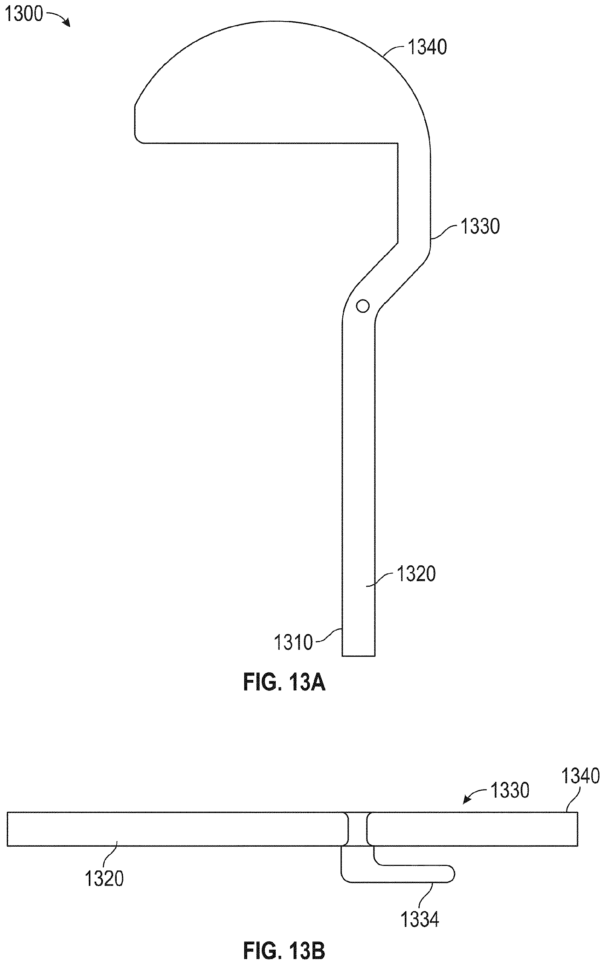

As depicted in Fig. 13A and 13B, in some embodiments, the tool 1300 may be formed from multiple pieces. For example, the body 1310 including a proximal end portion 1320 and a central portion 1330 extending generally longitudinally and a distal end portion 1340 extending generally transversely may be formed as a single first piece configured to be combined with a free arm 1334 second piece. The two or more pieces may be configured to matingly engage, via snapping, pressure fitting, screwing-in, buttons, magnets, and the like. The two or more pieces may alternatively be glued, swaged, crimped, US welded, over-molded, and the like. The free arm 1334 second piece may be secured to one or both of a top or bottom sides of the central portion 1330. A multiple piece tool of such design of Figs. 13A-13B, like the tool of Fig. 12A, may be configured to form either a right or left handed design as needed by simply adding the free arm second piece 1334 to the appropriate side of the body 1310 and possibly flipping the tool over 180 degrees, if needed.

The mesh delivery tools described herein are configured to be used with any implantable mesh configured to repair and/or treat soft tissue defects, such as hernias, prolapses, suture line reinforcements and the like. In some embodiments, the mesh is a self-gripping mesh including a plurality of grip-members positioned on at least one face thereof, the grip members configured to attach directly to tissue and/or other portions of the same or different mesh. In some embodiments, the mesh includes a slit with or without grip-members positioned thereon. In some embodiments, the tools described herein may be used with a ProGrip^{®} mesh (Medtronic).

In some embodiments, a method of repairing or treating a soft tissue defect such as a hernia is described and includes one or more of the following steps: introducing a surgical mesh in a folded configuration and secured at least in part to a mesh delivery tool as described herein into a patient; positioning the surgical mesh, via the mesh delivery tool, to a site of implantation; attaching at least a portion of the mesh to biological tissue at the site of implantation; freeing the mesh of the mesh delivery tool; and removing the mesh delivery tool from the patient.

The mesh delivery tool may include a body defining a length and a width, the length extending longitudinally between a proximal end portion and a distal end portion with a central slotted portion positioned therebetween, the width extending transversely between a first side and an opposite second side, the distal end portion including a fixed side part extending from the central slotted portion and a free side part opposite the fixed side part, the distal end portion extending between the first side and the second side to define the width of the body. The mesh may be a self-gripping mesh.

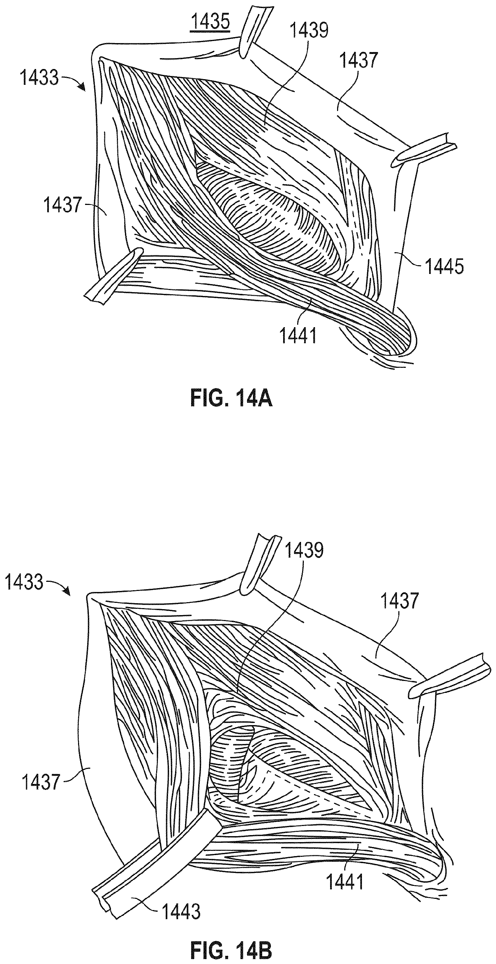

As shown in Figs. 14A-14F, the mesh delivery tools described herein may be used in combination with a self-gripping implantable surgical mesh to treat and/or repair an inguinal hernia. With reference to FIGS. 14A-14F, a method for treating and/or repairing an inguinal hernia using a mesh delivery tool as described herein 1400 and/or a surgical kit as further described hereinbelow. While the following description is provided for the repair of an inguinal hernia, the tool of the present disclosure is not limited to use in repairing only inguinal hernias.

The repair or treatment of an inguinal hernia in accordance with the procedure of the present disclosure may be performed by medical personnel directly (e.g., manually) or indirectly (e.g., via robotic assistance or a robotic surgical system). The procedure may be performed by an open approach and may begin with the cutting of a transverse oblique incision 1433 in a fold of a groin of a patient 1435. The incision may be between 1 cm and 10 cms, in some embodiments between 2 cms and 7 cms. Thereafter, a superficial inguinal space of patient 1435 is widely dissected, thereby freeing fascial surfaces 1437 and creating a surgical site 1439 (see FIG. 14A). Next, the spermatic cord 1441 of patient 1435 is mobilized using a latex band 1443 (see FIG. 14B).

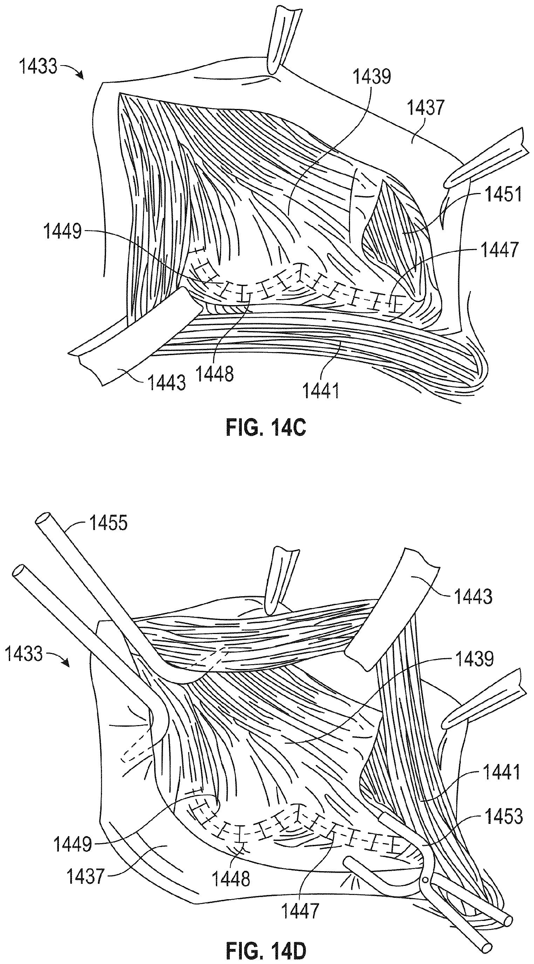

Medical personnel then identifies a hernia sac 1445 (indirect hernia) or a hernia bulge (direct hernia) and repairs the hernia. Thereafter, a relaxing incision 1451 in the internal oblique fascia is made to reduce tension in this area. For an indirect hernia, the internal inguinal ring may be tightened with an absorbable stitch. For a direct hernia, the hernia sac 1445 is imbricated to temporarily reduce the hernia bulge and then the superior and medial portion of the transverse layer is drawn downward and sutured 1448 to the superior pubic ligament 1447 and to the anterior femoral sheath 1449 (see FIG. 14C).

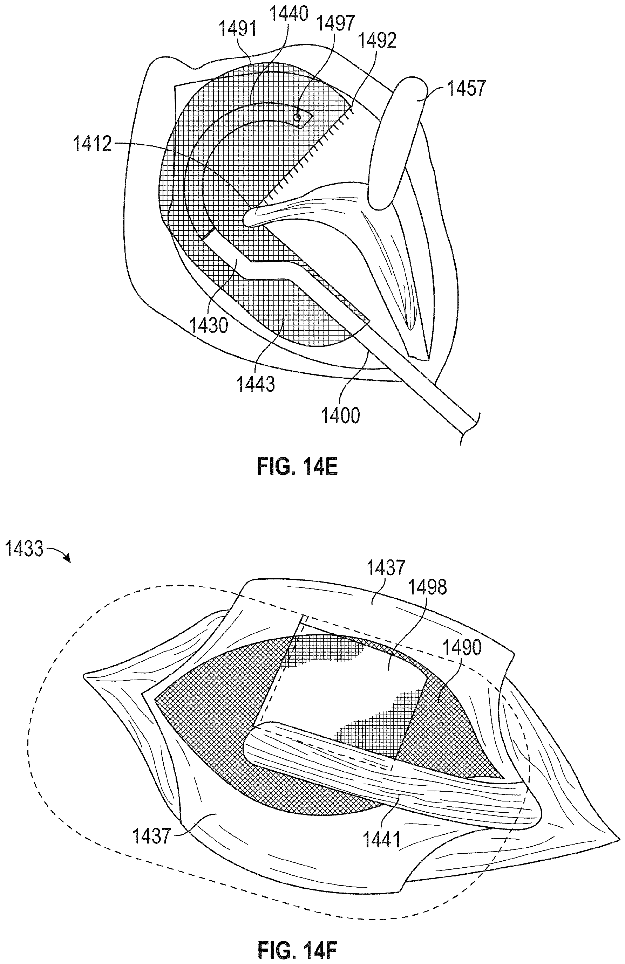

As shown in FIG. 14D, the size of surgical site 1439 is expanded using a flag retractor 1453 and a Gelpi retractor 1455. Then, tool 1400 of the present disclosure including a folded and/or overlapped mesh 1490 is positioned over the surgical site 1439 such that the distal end portion 1440 is positioned between the site 1439 and the mesh 1490, and in particular embodiments, the self-gripping grip members 1492 extending from the mesh 1490 (see FIG. 14E). The distal end portion 1440, the central slotted portion 1430, and the proximal handle portion 1420 of the delivery tool 1400 at least partially positioned around or circumferentially wrap around the spermatic cord 1441.

Once the mesh 1490 is in the correct position, pressure can be applied to the mesh 1490 via the tool 1400 forcing at least the grip-members 1492 around the outer periphery of the first flat portion 1491 of the mesh 1490 to begin to attach directly to the tissue. In addition, the fold second piece 1493 of mesh 1490 (including any flap) may be removed or freed of the central slotted portion 1430 and any fastening elements or sutures 1497 cut to free the mesh 1490 of the tool 1400. Next, the delivery tool 1400 may be removed from the site and the remainder of mesh 1490, including the second previously folded piece, may be unfolded, and properly positioned around spermatic cord 1441 with the cord generally passing through the center opening 1412 of the mesh 1490. In some instances, additional surgical fasteners, such as sutures, tacks, clips, staples, glues, etc. may be used along the border to assure mesh 1490 does not move.

The final step in positioning the mesh 1490 includes removing the Alice clamp 1457 and closing any flap 1498 around spermatic cord 1441, thus creating a new internal ring that is completely tension free and custom made (see FIG. 14F). The external fascial surfaces 1437 are then closed with another layer of surgical fasteners, and the skin is also closed in a similar manner.

Surgical kits are also provided herein including at least one of the mesh delivery tools described herein and an implantable surgical mesh. In some embodiments, the surgical kit includes the surgical mesh preloaded into the delivery tool (see Figs. 2A-2B) in order to limit or save preparation time and also limit or avoid the touching of the mesh prior to implantation. In some embodiments, the surgical kit includes the surgical mesh separate from and/or not preloaded into the delivery tool.

The mesh delivery tools described herein may be used with any conventional surgical mesh. In some instances, the surgical mesh may be free of gripping members. In some instances, the surgical mesh may further include a coating (e.g., porous, or non-porous coating) including a bioactive material (e.g., adhesive material, anti-adhesive material, therapeutic material, cellular material, etc.). The mesh delivery tools described herein may be used to deliver a coated surgical mesh into a patient without the coating on a first side of the mesh interacting with a second opposite side of the mesh and/or body fluids/tissue inside the patient during delivery.

It will be understood that various modifications may be made to the embodiments disclosed herein. Thus, those skilled in the art will envision other modifications within the scope and spirit of the disclosure.

## Claims

1. A mesh delivery tool comprising:
a body defining a length and a width, the length extending longitudinally between a proximal end portion and a distal end portion with a central slotted portion positioned therebetween, the width extending transversely between a first side and an opposite second side, the distal end portion extending between the first side and the second side defining the width of the body, the distal end portion including a fixed side part and a free side part opposite the fixed side part, the fixed side part extending from the central slotted portion.

2. The mesh delivery tool of claim 1, wherein the proximal end portion defines a generally straight rod extending along a longitudinal axis at a location ranging from a center of the width to one of the first or second sides opposite the free side part of the distal end portion.

3. The mesh delivery tool of claim 2, wherein the location of the proximal end portion is on the center of the width and the central slotted portion defines a bent rod extending from the proximal end portion to the fixed side part of the distal end portion.

4. The mesh delivery tool of claim 2, wherein the location of the proximal end portion is off-set from the center of the width and closer to the first side opposite the free side part of the distal end portion and the central slotted portion defines a bent rod extending from the proximal end portion to the fixed side part of the distal end portion.

5. The mesh delivery tool of claim 1, wherein the location of the proximal end portion is on the first side and the central slotted portion is a generally straight rod extending from the proximal end portion to the fixed side part of the distal end portion, the body defining a general sickle shape.

6. The mesh delivery tool of claim 1, wherein the distal end portion defines a bent rod extending transversely between the first side and the second side.

7. The mesh delivery tool of claim 6, wherein the free side part of the distal end portion further comprises a tab member extending transversely back towards the fixed side part of the distal end portion, longitudinally towards the proximal end portion, or both.

8. The mesh delivery tool of claim 7, wherein the tab member extends transversely back completely to the fixed side part of the distal end portion forming a frame defining a distal aperture.

9. The mesh delivery tool of claim 1, wherein the body defines a generally question-mark shape.

10. The mesh delivery tool of claim 1, wherein the central slotted portion comprises a first slot separating a central body rod and a first free central arm along the central slotted portion, the central body rod secured to both the proximal and distal end portions of the body, the first free central arm secured to the body on only a proximal part, wherein the first free central arm, the first slot, and the central body rod are vertically aligned and the first slot is configured to receive and maintain at least a flap or folded portion of a mesh therein.

11. The mesh delivery tool of claim 10, wherein the first slot extends only through the central slotted portion of the body.

12. The mesh delivery tool of claim 10, wherein the first slot extends through the central slotted portion and at least a portion of one of the proximal end portion, the distal end portion, or both.

13. The mesh delivery tool of claim 10, wherein the first slot includes a textured or micro-textured surface configured to engage the mesh when positioned therein.

14. The mesh delivery tool of claim 10, wherein the first slot includes a one or more non-linear edges.

15. The mesh delivery tool of claim 10, further comprising a hinge proximal the first slot, the hinge configured to allow the central rod and the first central arm to pivot between an open and closed configuration.

16. The mesh delivery tool of claim 10, wherein the central slotted portion further comprises a second slot separating the central body rod and a second free central arm along the central slotted portion, the second free central arm secured to the body on only a proximal part thereof, wherein the second free central arm, the second slot, and the central body rod are vertically aligned and the second slot is configured to receive and maintain at least a flap or folded portion of a mesh therein.

17. The mesh delivery tool of claim 1, wherein the body is a one-piece tool.

18. The mesh delivery tool of claim 1, wherein the distal end portion of the body further comprises one or more fastener openings defined therethrough, the fastener opening configured to allow a fastener to pass therethrough to secure a portion of a mesh to the body.

19. A surgical kit comprising:
a mesh delivery tool including a body defining a length and a width, the length extending longitudinally between a proximal end portion and a distal end portion with a central slotted portion positioned therebetween, the width extending transversely between a first side and an opposite second side, the distal end portion including a fixed side part extending from the central slotted portion and a free side part opposite the fixed side part, the distal end portion extending between the first side and the second side to define the width of the body; and
an implantable surgical mesh.

20. The surgical kit of claim 19, wherein the implantable surgical mesh includes one or more of a slit extending to an outer periphery of the mesh, a plurality of grip members extending from at least one face of the mesh, or both.

21. A method of repairing or treating a soft tissue defect comprising:
introducing a surgical mesh in a folded configuration and secured at least in part to a mesh delivery tool into a patient, the mesh delivery tool including a body defining a length and a width, the length extending longitudinally between a proximal end portion and a distal end portion with a central slotted portion positioned therebetween, the width extending transversely between a first side and an opposite second side, the distal end portion including a fixed side part extending from the central slotted portion and a free side part opposite the fixed side part, the distal end portion extending between the first side and the second side to define the width of the body; and
positioning the surgical mesh, via the mesh delivery tool, to a site of implantation;
attaching at least a portion of the mesh to biological tissue at the site of implantation; and
freeing the mesh of the mesh delivery tool; and
removing the mesh delivery tool from the patient.
